# EUROPEAN PATENT APPLICATION

(11) **EP 4 438 083 A1**
(43) Date of publication of application: **02.10.2024**
(21) Application number: 23164208.3
(22) Date of filing: 27.03.2023
(51) Int. Cl.: A61M 5/315, A61M 5/20, A61M 5/32

(54) **AN ELECTRONIC MODULE**

(71) Applicant: Ypsomed AG, 3401 Burgdorf (CH)
(72) Inventor: Hulliger, Manuel, 4563 Gerlafingen (CH); Mangold, Stefan, 3097 Liebefeld (CH); Zumstein, Dominik, 3014 Bern (CH); Bosshard, Simon Martin, 3324 Hindelbank (CH); Schrul, Christian, 3400 Burgdorf (CH); Etter, Leonie, 3011 Bern (CH)
(74) Representative: Meier Obertüfer, Jürg

(57) **Abstract**

An electronic module configured for releasable attachment to a medicament delivery device (20) comprising a printed circuit board (16c) with an electronic circuit (29), a battery (16b), and a switch for activating the electronic module (16). The switch comprises an electric activation contact (28) providing a connection with the electronic circuit (29) and a battery contact extension (27) extending from a terminal (16d, 16e) of the battery (16b). The battery contact extension (27) is biased towards the electric contact (28) for closing a switching gap (30) between the battery contact extension (27) and the electric activation contact (28), and the battery contact extension (27) is configured to engage an actuation element (11, 6) of the medicament delivery device (20) such that the actuation element (11, 6) pushes the battery contact extension (27) against the bias thereby creating the switching gap (30).

## Description

### TECHNICAL FIELD

The current invention relates to an electronic module configured for attachment to a medicament delivery device including a switch for activating the electronic module.

### BACKGROUND OF THE INVENTION

Injection or infusion devices such as injections pens, auto injectors, autopens, patch injectors or patch pumps, use a drive mechanism for expelling medicament from the device. The drive mechanism includes mechanical components for advancing, for example, a piston rod forwarding a bung in a reservoir or to drive a pumping mechanism. The injection or infusion devices may be disposable or reusable devices. These devices are mainly mechanically driven using springs or are driven by gas that is either generated in-situ or compressed in a cartridge. These mechanical devices have features for providing feedback to the user such as a start of injection click or an end of injection click generated by the drive mechanism. The monitoring functions of these devices for providing feedback to the user are limited by the mechanical drive mechanism used.

There is a need to include additional monitoring and communication features to those mechanical devices for example by adding electronic modules to the mechanical device. Examples for those electronic modules may be a connectivity module with a transmitter and/or receiver to allow communication with other devices, a sensing module to allow for sensing patient parameters such as blood glucose values, a location module indicating geographical locations, a timer or calendar module, a communication or training module including a loudspeaker, or a sensing module to detect impact. The electronic module may be added as an add-on or supplementary device to the existing mechanically driven device. The add-on may be a separate add-on that the end user attaches to the injection or infusion device or the add-on is integrated into the mechanical device by the device manufacturer, a so called integrated add-on that may be removed after use.

The electronic module is configured to monitor functions of the device such as, for example, the start of the injection, the end of injection, the holding time after injection, the removal of the electronic module, the re-attachment of the electronic module to another medicament delivery device after being used on a medicament delivery device, monitoring and counting the number of times the monitoring unit has been attached to a plurality of devices ( lifetime management), the identification of the medicament used, and the like. The data may be stored in a storage module of the electronic module and the electronic module may be configured to transmit the data to an external device or to receive information from an external device.

The electronic module needs to be powered by a battery and the service lifetime of the module may be restricted by the battery capacity and/or the time that the electronic module is active or at least a part of the electronic module is active. The electronic module may be activated to switch-on the electronic module or a part of the electronic module may be activated for monitoring functions of the medicament delivery device. The electronic module therefore needs a switch for activating and/or actuating the electronic module.

In EP 4122512 A1 an autoinjector is disclosed with a module housing closing an electronic module that is releasable attached to a main housing including the injection mechanism. The electronic module includes a separate switch attached to a printed circuit board (PCB) for actuating the module thereby requiring additional components that consume space in the module.

WO2017032586 presents a monitoring unit for a medicament delivery device with an actuation switch. The actuation switch is a separate switch positioned on the PCB thereby requiring additional components and additional length of the monitoring unit.

It is an object of the present invention to improve the electronic module over the prior art. There is a need for a robust and reliable electronic module requiring less components allowing for space saving configurations. These objectives are solved by the independent claim and specific variants are disclosed in the dependent claims. Furthermore a medicament delivery device including the electronic module is presented.

### DEFINITIONS

The term "medicament" or "medication" includes any flowable medical formulation suitable for controlled administration through a means such as, for example, a cannula or a hollow needle and includes a liquid, a solution, a gel or a fine suspension containing one or more medical active ingredients. A medicament can be a composition including a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "a battery extension" does not exclude the fact that there may be two battery extensions that functionally or structurally fulfill the purpose of "a battery extension". The mere fact that certain elements or steps are recited in distinct claims shall not preclude the existence of further meaningful combinations of these elements or steps.

### GENERAL DESCRIPTION OF THE INVENTION

The current invention relates to an electronic module configured for releasable attachment to a medicament delivery device. The electronic module includes a printed circuit board (PCB), an electronic circuit being located on the printed circuit board, a battery and a switch for actuating the electronic module. The switch includes an electric activation contact that is part of and connected to the electronic circuit and a battery contact extension extending from, and contacting a terminal of the battery. The battery contact extension is biased towards the electric activation contact for closing a switching gap between the battery contact extension and the electric activation contact thereby activating the switch. The battery contact extension is configured for engaging an actuation element of the medicament delivery device such that the actuation element pushes the battery contact extension against the bias thereby creating the switching gap and opening the switch.

An electronic module is presented that is configured for releasable attachment to a medicament delivery device including a printed circuit board with an electronic circuit, a battery and an activation contact providing a connection to the electronic circuit. A battery contact extension extends from a terminal of the battery, the battery contact extension is biased towards the activation contact for closing a switching gap between the battery contact extension and the electric activation contact. The battery contact extension is configured to engage an actuation element of the medicament delivery device such that the actuation element may push the battery contact extension against the bias thereby opening and closing the switching gap thereby forming a switch.

The electronic module is configured for releasable attachment and/or releasable detachment from the medicament delivery device. The electronic module may be releasable attached to the same or another medicament delivery device. The same medicament delivery device and the another medicament delivery preferably include the same delivery mechanism or same mechanical interface for connecting the electronic module. The medicament delivery device may be an injection device, an auto injector or a patch injector.

The printed circuit board may be rigid, flexible or include flexible and rigid elements. The combination of rigid and flexible elements may allow for stacking of the rigid PCB elements on top of each other for a space saving configuration of the PCB in the electronic module. The battery may be rechargeable or non-rechargeable for powering the electronic module. The battery is preferably connected to the PCB and may be provided in a battery holder. The battery holder may be mechanically connected to the PCB or may be fixated to a part of the housing of the electronic module, for example to a closure cap or housing of the electronic module. Further components of the electronic module that may be located on the PCB include a microprocessor, a transmitter, a receiver, an antenna, a storage module, a communication module for the user or a scanner or camera. The communication module may include a visual display such as a LCD or OLED display, a loudspeaker, or buzzer. The scanner may be configured to read bar codes or 2-dimensional QR codes. The electronic module may include an input device such as a touch sensitive screen.

The actuation of the switch may activate or wake up the electronic module or activate the microprocessor located on the PCB. Actuation of the switch may also de-activate the electronic circuit or de-activate a part of the electronic circuit.

The switch includes an electric activation contact or contact area on, or that is part of the electronic circuit. One end of the battery contact extension contacts the terminal of the battery or contacts a battery holder holding the battery. The battery contact extension may also be part of, or depend or exend from the battery holder holding and contacting the battery. The battery contact extension is biased towards the electric activation contact or contact area and configured to close the switching gap between the battery contact extension and the electric activation contact and close the switch. The battery contact extension is configured to engage the actuation element of the medicament delivery device which may be a drive element or a signaling element or a display element. The actuation element of the medicament delivery device is configured to push on, or axially displace at least a part of the battery contact extension against the bias thereby configured to create the switching gap and opening the switch. The switch is open or not actuated. Compared to the prior art, an additional switch and activation element of the medicament delivery device may not be required such that the electronic module can be actuated with less components and represents a space saving configuration. The space may be used, for example, for a battery with a larger capacity.

Upon release of the electronic module from the medicament delivery device, the switch may be actuated as there is no engagement between the actuation element and the battery contact extension and the bias on the battery contact extension closes the switch when the user removes the electronic module from the medicament delivery device.

When the electronic module is attached to the medicament delivery device then the switch may be actuated by the delivery mechanism of the medicament delivery device. The switch may be actuated several times and each time the activation of the switch may trigger a signal different from the previously generated signal by the electronic module. The switch may be actuated upon attachment of the module or may be actuated when the medicament delivery device is used during medicament delivery or may be actuated when the electronic module is removed. The switch may monitor several functions or states of the medicament delivery device throughout its service life.

The electronic circuit or the microprocessor in the electronic circuit or the receiver/transmitter may be activated by the switch when the switching gap is closed for a defined minimum closure time. Wake up of the electronic circuit may be established when the contact is established (switch is closed) for a certain duration, for example more than 10 milliseconds, preferably more than 100 milliseconds. Closing the switch may initially start the wake up procedure for the microprocessor but if not followed by a continued actuation of the switch then the electronic module may go back in the sleeping mode. This may avoid unintended actuation, for example during impact (drop-test) where the impact results in a short closure time of the switch.

The terminals of the battery may be both in permanent contact with the PCB thereby powering the electronic module or powering a part of the electronic circuit permanently. Another part of the electronic circuit may remain in a sleeping mode. The part of the electronic circuit containing power consuming components such as the microprocessor, or the transmitter may initially not be powered by the battery. Actuation of the switch may act as a load switch thereby adding and powering further components of the electronic circuit. Alternatively, one of the terminals of the battery may be in contact with the PCB and the other terminal may be connected to the PCB via the switch, in this case the electric power is not supplied to the electronic circuit until the switch is operated thereby activating the electronic module.

The connectors between the terminals of the battery and the PCB may have a combined electrical (conductive) function and also provide mechanical support to position the battery or the battery holder on the PCB.

The battery contact extension of the electronic module may include a spring element or spring biasing the battery contact extension towards the electric activation contact. The spring element or spring may be a coil spring, a torsional spring or a leaf spring. The spring may be attached to, or may be part of the battery holder.

The battery contact extension may include a detection pin and the spring element or spring is located between the terminal of the battery and the detection pin. The spring may also be located between the battery holder and the detection pin. The detection pin may be at the distal end of the battery contact extension and may extend from the spring element.

The actuation element of the medicament delivery device may be configured to abut the detection pin or the distal end of the detection pin of the battery contact extension when the electronic module is attached to the medicament delivery device thereby creating the switching gap or opening the switch.

The detection pin and the spring element may be provided as a unitary component and made from an electrically conductive material such as a metal. Examples are stainless steel or cupper. The switch may be configured as a bi-stable clicker element having two preferred configurations one for the closed switch and one for the open switch. The switch may be made from a sheet, a wire or is a stamped part. The battery holder, the spring and the detection pin may be made as a unitary component thereby reducing the number of components to be used for the electronic module.

The battery holder holds the battery and contacts the terminals of the battery and the battery holder may be mechanically fixed to the PCB or to another housing part. The contact between the PCB and the battery or battery holder may provide mechanical support only, or may provide an electric activation contact only or may provide both mechanical support and an electric activation contact.

In a preferred embodiment, the spring element is a leaf spring and the detection pin may extend from the leaf spring and is oriented essentially perpendicular to the leaf spring forming a kink or bend between the leaf spring and the detection pin. Essentially perpendicular is defined as an angle between eighty and one hundred degrees, preferably between eighty five and ninety five degrees. Alternatively, the detection pin is tilted to the leaf spring with an angle ranging between forty five and eighty degrees or an angle between one hundred and one hundred forty five degrees. The spring may be formed by the kink between the leaf spring and the detection pin.

The electric activation contact on the electronic circuit may at least partially surround a passage or boring in the printed circuit board. The electric activation contact may be made from gold or copper and may be integrated in, and connected to the conductive paths of the electronic circuit. The electric activation contact surrounding the passage is preferably on one side of the PCB. Alternatively, the electric activation contact is on both sides of the PCB.

The detection pin may at least partially pass or go through the passage and the distal end of the detection pin is configured to engage the actuation element of the medicament delivery device. Preferably, the electric activation contact is on one side of the passage and the distal end of the detection pin at the opposite side of the passage.

The kink may be configured for contacting the electric activation contact of the electronic circuit on one side of the passage.

The passage in the PCB may have a fixed dimension, for example a fixed diameter or may have a variable diameter with respect to the central axis of the passage. The passage may be tapered. The distal section of the passage may include a smaller dimension for (mechanically) guiding the detection pin through the passage whereas the proximal part has a greater dimension (diameter) for the electric activation contact. Correct guidance for the battery contact extension may increase the reliability, robustness and impact resistance for the switch. A greater dimension of the entrance section of the passage for receiving the detection pin may be beneficial for reducing the sensitivity of the switch to tolerance variations during manufacturing.
The PCB may have a plurality of passages, one passage for guidance of the battery contact extension and another passage may provide the electrical switching when closing the switching gap. The battery contact may have a plurality of battery contact extensions.

The kink may be located at one side of the PCB for contacting the electric activation contact and the distal end of the detection pin may be at opposite side of the PCB for contacting the actuation element.

The electric activation contact may at least partially cover the inner surface of the passage. The contact area may be increased from a top surface of the PCB where the electric activation contact at least partially surrounds the passage, to including an inner surface of the passage thereby increasing the reliability of the switch.

The battery contact extension may include a radial or lateral extension and/or a tapered section for contacting the electric activation contact wherein the radial or lateral extension has an outer dimension greater than the inner dimension of the passage. The inner dimension may be the diameter of the passage. The radial or lateral extension may be shaped as a flange. The tapered section may depend from the lateral extension or flange. The radial or lateral extension may be adjacent to the kink and the radial or lateral extension may be positioned between the kink and the tapered section. The radial or lateral extension is configured for contacting the electric activation contact of the electronic circuit surrounding the passage whereas the tapered section is configured for engaging the contact surface inside the passage of the PCB. The tapered section and/or the flange may remove contamination or a corroded surface from the electric activation contact upon closure of the switch. The tapered section may be roughened to act as a sandpaper thereby removing a corrosion layer from the conductive surface that may have built up during shelf life.

The actuation element of the medicament delivery device may axially move along, or tilt with respect to, or rotate around a longitudinal axis of the medicament delivery device when the medicament delivery device is used. When an electronic module is attached to the delivery device, then the actuation element may release the engagement between the actuation element and the battery contact extension and close the switching gap and actuate the switch. The switch may be repeatedly actuated during use of the medicament delivery device, for example upon attachment of the module to the medicament delivery device or at the start and/or at the end of the injection. When the medicament delivery device is removed after use, the switching gap may be closed again as the actuation element is not in contact with the battery extension. Each time the mechanical actuation of the switch may activate the electronic module or a part of the electronic module. As an alternative, actuation may de-activate the electronic module or a part of the electronic module.

In another embodiment, the actuation element of the medicament delivery device indirectly actuates the switch via a coupling part or coupling member which may axially move, tilt or rotate due to the movement of the actuation part during dose delivery and the coupling part or coupling member may abut the battery contact extension, preferably the detection pin.

The electronic module may include a mechanical interface that is configured for releasable attachment to or releasable detachment from a complementary mechanical interface on the medicament delivery device. The mechanical interface includes couplers or coupling means for engaging complementary couplers or coupling means on the complementary mechanical interface of the medicament delivery device. The mechanical interface may be configured for repeated attachment or repeated detachment, or the mechanical interface of the electronic module may only be coupled once to the complementary mechanical interface on the medicament delivery device. The electronic module may, after detachment, not be attached to an (empty) device and may only be attached to a ready to use or full medicament delivery device. This may promote that the therapy may only be started with a full medicament delivery device.

The detection pin of the switch may be part of or located in the mechanical interface of the electronic module. Alternatively, the switch is located outside the mechanical interface.

A medicament delivery device may include the electronic module described above. The electronic module may be attached to the medicament delivery device and the assembly may be used by the user. The battery contact extension or the detection pin may engage the actuation element of the medicament delivery device during attachment of the electronic module thereby opening the switch and creating the switching gap against the bias of the spring. The mechanical interface of the electronic module engages a complementary mechanical interface on the medicament delivery device during attachment. The mechanical interface of the electronic module may have arms, elastic arms, protrusions, releasable snap fit connectors, a screw type of connector, a bayonet type connector a push-and-rotate type of connector configured to engage the complementary mechanical interface on the medicament delivery device including recesses rims, elastic arms, screw type of connectors or the counterparts for the bayonet connector or push-and-rotate-type of connectors. The mechanical interface of the electronic module may be configured to lock or unlock the medicament delivery device, e.g. the medicament delivery device may only be operated in an unlocked state with an attached electronic module.

The medicament delivery device may be a spring driven auto injector including an injection spring for moving a drive element in the distal direction during dose delivery. The injection spring may rotate the actuation element or tilt the actuation element or axially move the actuation element in the distal direction to release the engagement between the battery contact extension and the actuation element thereby closing the switch. During an injection, the actuation element may move in the proximal direction after first being moved in the distal direction thereby actuating the switch again.

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

### LEGENDS TO THE FIGURES

- Figure 1:: Exploded view of an autoinjector,
- Figure 2a:: Longitudinal section of the autoinjector in Figure 1,
- Figure 2b:: Longitudinal section of the autoinjector, in a view perpendicular to Figure 2a,
- Figure 3a:: Longitudinal section of an electronic module according to the prior art with a separate switch and detection pin; autoinjector before use,
- Figure 3b:: The electronic module according to the prior art of Figure 3a; autoinjector during use,
- Figure 4a:: Longitudinal section of an electronic module according to an embodiment of the current disclosure; autoinjector before use,
- Figure 4b:: The electronic module of Figure 4a; autoinjector during use,
- Figure 5a:: Perspective view of the electronic module; autoinjector before use,
- Figure 5b:: Perspective view of the electronic module, autoinjector during use,
- Figure 6a:: Side view of the electronic module, switch open,
- Figure 6b:: Side view of the electronic module, switch closed,
- Figure 7a:: Perspective view of an electronic module with a tapered section for the battery contact extension; switch is open,
- Figure 7b:: Perspective view of an electronic module with a tapered section for the battery contact extension; switch is closed,
- Figure 8a:: Detail of Figure 7a,
- Figure 8b:: Detail of Figure 7b.

### DETAILED DESCRIPTION OF THE FIGURES

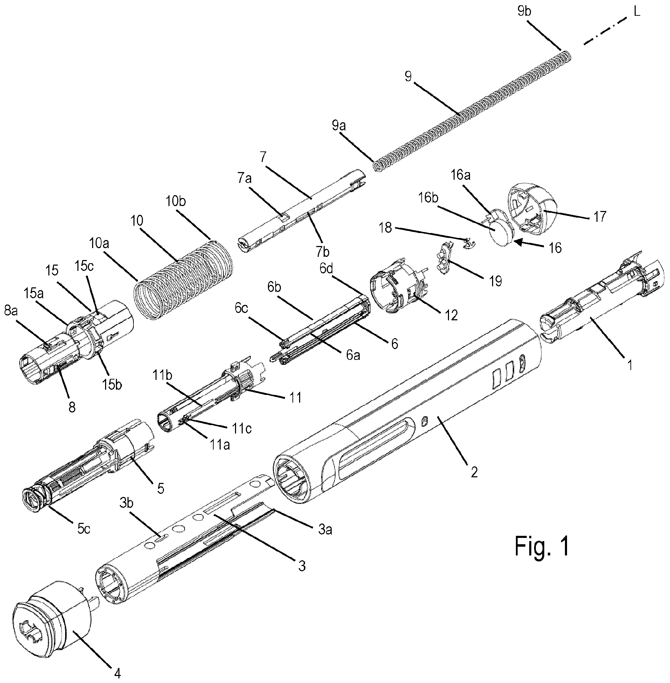

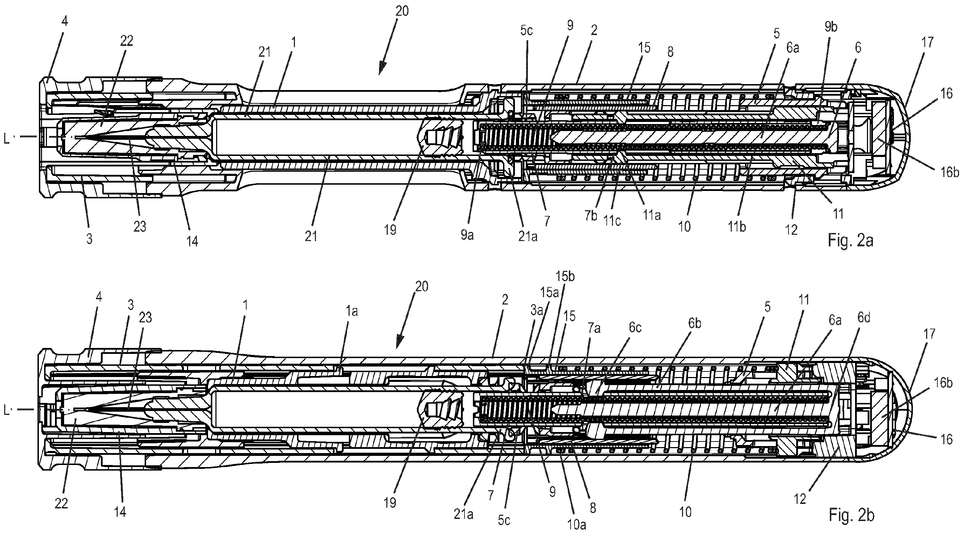

Figure 1 presents an exploded view of an autoinjector. An elongated sleeve shaped housing 2 can be gripped by the user during an injection and the housing defines a longitudinal axis L. An injection mechanism that is configured to be driven by a coiled compression spring is enclosed in the housing 2. To illustrate the function of the components, Figures 2a and 2b depict longitudinal sections of the autoinjector 20. A prefilled syringe 21 (Figure 2) is received in a syringe holder 1 and the syringe holder 1 is axially and rotationally secured to the housing 2 with a snap-fit connection. The autoinjector 20 as delivered to the patient is closed at the distal end by a pen cap 4 that must be removed before using the autoinjector. A needle shield 22 of the prefilled syringe 21 is coupled to the cap 4 by a needle shield remover 14 such that removal of the pen cap 4 also removes the needle shield 22 thereby exposing hollow needle 23. A needle cover sleeve 3 is axially guided by, and can be moved relative to the housing 2 along the longitudinal axis L a distance corresponding to an actuation hub. The needle cover sleeve 3 can slide in the proximal direction for actuating the injection mechanism to expel medicament from the autoinjector 20. A mechanic holder 5 is snap-fitted into and rotationally and axially secured to the housing 2. The mechanic holder 5 includes an elastic element 5c (holding spring) at the distal end which abuts the proximal end 21a of the prefilled syringe 21 and biases the prefilled syringe distally into the syringe holder 1.

The injection mechanism includes a compression spring acting as an injection spring 9. The injection spring 9 in its initial state is almost completely enclosed by a piston rod 7 and the distal end 9a of the injection spring 9 biases the piston rod 7 into the distal direction. The proximal end 9b of the injection spring abuts a holding element 6 and the holding element 6 includes two arms 6b extending from a proximal end 6d in the distal direction and one central pin 6a for guiding the injection spring 9. Each arm 6b includes a protrusion 6c directed towards the axis L and both protrusions engage recesses 7a on the outer surface of the piston rod 7. In the initial state of the autoinjector, a switching module 8, 15 including switching sleeve 15 and a locking sleeve 8 prevent a deflection of the arms 6b as the arms 6b are confined within the locking sleeve 8 and therewith prevent a relative movement between the piston rod 7 and the holding element 6 thus keeping the injection spring 9 in a compressed state. The piston rod 7 is prevented from movement into the distal direction. At least the distal end 15a of the switching sleeve 15 is engaged or abuts with a proximal end 3a of the needle cover sleeve 3. The switching sleeve 15 is biased by a needle cover sleeve spring 10 into the distal direction. The distal end 10a of the needle cover sleeve spring 10 abuts a rim 15b on the switching sleeve 15 and the proximal end 10b of the needle cover sleeve spring 10 abuts a signaling element 11 and the signaling element abuts an end cap 12 that is axially and rotationally secured to the housing 2. In the initial state of the autoinjector 20, the force of the needle cover sleeve spring 10 in the proximal direction is guided to the end cap 12 of the housing 2 via the signaling element 11. In the distal direction, the needle cover sleeve spring 10 biases the needle cover sleeve 3 into the distal direction via the switching sleeve 15. For starting the injection, the needle cover sleeve 3 is pushed against the skin of the patient and moved in the proximal direction with respect to the housing 2 thereby moving the switching module 15, 8 in the proximal direction and tensioning the needle cover sleeve spring 10. In the most proximal position of the switching module 15, 8, the locking sleeve 8 may be axially engaged with the mechanic holder 5 (or alternatively to the signaling element 11 as will be discussed below) whereas the switching sleeve 15, axially guided and splined onto the locking sleeve 8, is kept by the needle cover sleeve 3 in the proximal position against the bias of the spring force provided by the needle cover sleeve spring 10.

The proximal movement of the switching module 8, 15 releases the engagement between the protrusions 6c on the holding element 6 and the recesses 7a in the piston rod 7. The spring force is released and the piston rod 7 moves into the distal direction whereas the holding element 6 can move an initial hub in the proximal direction until the proximal end 6d of the holding element 6 abuts the end cap 12 of the housing 2. Optionally, an acoustic or tactile signal is generated upon abutment indicating the start of the injection. The piston rod 7 abuts a bung 19 in the prefilled syringe which is moved distally towards the outlet thereby expelling medicament through the needle 23.

The needle cover sleeve spring 10 is a metal coil spring acting as a compression spring and the proximal end 10b abuts the signaling element 11 which abuts the end cap 12 of the housing 2 before injection. The signaling element 11 includes two longitudinally extending arms 11b each having a protrusion 11a oriented towards the longitudinal axis L. The protrusions 11a engage another recesses 7b in the piston rod 7 and the sleeves of the switching module 8, 15 ensure that the signaling element 11 is axially coupled to the piston rod 7 before and during the initial stage of the injection. The arms 11b with protrusions 11a are also confined within the locking sleeve 8 thereby preventing release of the protrusions from the recesses. During the initial stage of the injection, the piston rod 7 is advanced distally and thereby also drives the signaling element 11 in the distal direction away from the end cap 12 through the locking sleeve 8. The signaling element 11 is moved in the distal direction by a distance or tensioning hub until the protrusions 11a are released from the recesses 7b in the piston rod 7 as the arms 11b are not confined by the locking sleeve 8 anymore and can flex radially outward. The arms 11b have another protrusion 11c that is directed in the radial outward direction and those outward protrusions 11c engage the switching module 8, 15 keeping the tensioning hub of the signaling element 11. The arms 11c preferably engage the locking sleeve 8 thereby keeping the locking sleeve 8 in the proximal position (alternatively the locking sleeve engages the mechanic holder 5 as discussed above). The arms 11b can slide along the outer surface of the piston rod 7 and at the end of the injection, the piston rod has advanced such that the arms 11b of the signaling element 11 can flex radially inward again and the signaling element 11 is released from the switching module 8, 15. The signaling element 11 is biased proximally by the needle cover sleeve spring 10 and is accelerated and moved back towards the end cap 12 thereby producing an audible and/or tactile end-of-injection click.

After the medicament has been delivered, the autoinjector 20 is removed from the skin and the switching sleeve 15 together with the needle cover sleeve 3 are forwarded by the needle cover sleeve spring 10 that is at least partially decompressed. The switching sleeve 15 moves in the distal direction and is sliding along the locking sleeve 8 which has been locked before injection to the mechanic holder 5. The switching sleeve 15 may be locked in the most distal position by a flexible arm 8a of the locking sleeve 8 engaging a cut out 15c of the switching sleeve 15 thereby preventing proximal movement of the switching sleeve 15 after injection. The needle cover sleeve 3 may be locked in the most distal position by arms 1a of the syringe holder 1 that radially extend into recesses 3b of the needle cover sleeve 3 thereby preventing proximal movement of the needle cover sleeve 3 after injection.

The end cap 12 is axially and rotationally fixed to the housing 2. The end cap 12 may be snap fitted, adhesively connected or welded to the housing 2. The end cap 12 can be the closure cap for the autoinj ector delimiting the mechanical components of the device in the proximal direction if there are no further modules added to the autoinjector. The end cap may provide protrusions, ribs or 3D structures acting as shock absorbers. Preferably, the end cap 12 provides a platform or adapter for adding an additional module to the autoinjector. Preferably this is an electronic module 16 and the module is closed by a closure cap 17 engaging the end cap 12 and/or housing 2 of the autoinjector. The closure cap 17 may provide protrusions, ribs or 3 D structures on the outer surface acting as shock absorbers. Preferably, the module is a releasable module that can be released and separated from the autoinjector after injection. The electronic module 16 may include a sensor or switch 16a and a battery 16b and a printed circuit board 16c. The printed circuit board 16c may include a microprocessor, a transmitter/receiver unit, an antenna and/or a light source such as a LED. The light source may be used to signal the status of the device to the user, for example "ready to use" or "injection completed". The sensor 16a may be part of the PCB 16c as well and is configured to detect if the signaling element 11 is in the most proximal position. The sensor 16a may be a switch located on the PCB 16c detecting whether the signaling element 11 is in its most proximal position before injection and/or the most proximal position after injection and/or the absence of contact to the sensor during an injection. The signaling element 11 may have a protrusion extending into the proximal direction and optionally guided through a passage in the end cap 12 for contacting the sensor 16a. Alternatively, the signaling element 11 indirectly contacts the sensor 16a, for example via a pivoting element, a rotating element or an element that moves along the pen axis 18 that is part of or coupled to the end cap 12. The microprocessor detects the signals from the sensor 16a and is capable to transmit the data (for example time, duration, failure) of an injection to an external device using a communication module including, for example a transmitter and/or receiver.

The signaling element 11 or an extension of the signaling element 11 may therefore contact the sensor 16a directly or indirectly before an injection as the signaling element is in the most proximal position. During the injection the signaling element is driven by the piston rod 7 in the distal direction and the signaling element 11 or an extension therefrom may not contact the sensor 16b. At the end of the injection, the signaling element 11 moves in the proximal direction and may contact the sensor 16b again. Each event may be recorded and may be transmitted to an external device or may trigger a visual or acoustic signal by the electronic module.

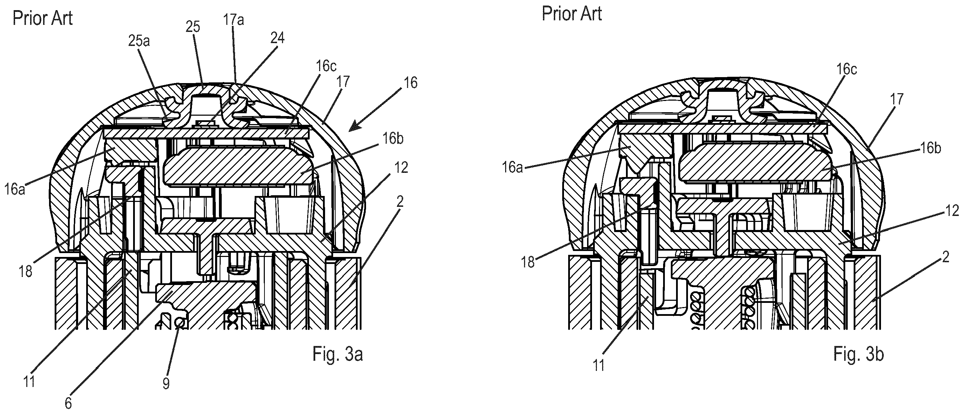

Figures 3a and 3b represent a detail of the electronic module 16 according to the prior art and includes a separate switch 16a on printed circuit board 16c. The electronic module is located between end cap 12 and closure cap 17. The electronic module includes the battery 16b that is connected to the PCB. The closure cap 17 includes an aperture 17a that is closed by a transparent plug 25 such that an LED light source 24 on the PCB can provide optical signals to the user. The plug 25 may include elastic arms 25a surrounding a bore in the plug for guiding the LED light. The elastic arms 25a may also provide a resilient force on the surface of the PCB for mechanically fixating the PCB or for compensating manufacturing tolerances. The electronic module 16 is attached to the proximal end of the auto injector by a mechanical interface, for example by a bayonet type of connection between the closure cap 17 and the housing 2 or between the closure cap 17 and the end cap 12. The housing 2 encloses the injection mechanism driven by injection spring 9 that may act on the signaling element 11 and/or the holding element 6 during different stages of the injection. In the embodiment presented in Figures 3a and 3b, the signaling element 11 is configured to abut the distal end of detection pin 18 (Figure 3a). During an injection, the signaling element 11 moves in the distal direction and actuates the switch 16a (Figure 3b). The detection pin in this embodiment moves along the longitudinal axis of the autoinjector but may also tilt or pivot with respect to the longitudinal axis L. The actuation of switch 16a may register the start of the injection or the end of the injection or trigger the start of a holding time after the injection. The electronic module according to the prior art presented in Figures 3a and 3b includes a separate switch 16a located on the PCB which occupies space, additionally a separate detection pin is used that is part of, and enclosed in the autoinjector.

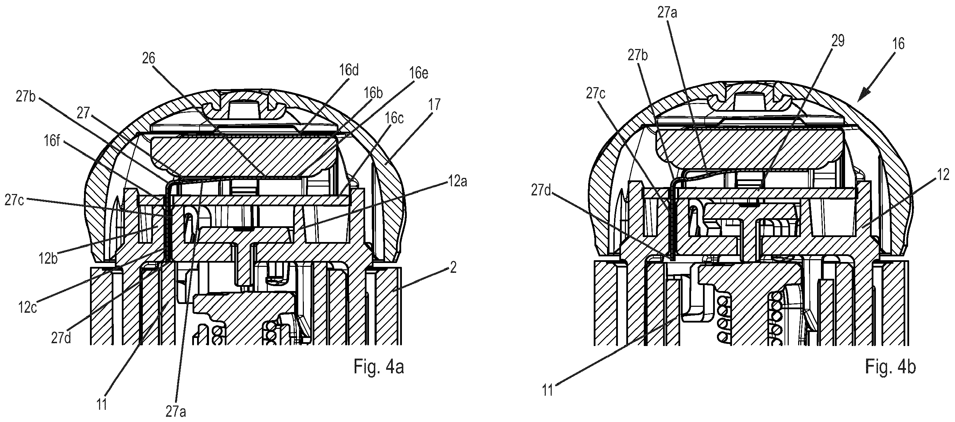

Figures 4a presents an electronic module according to the present invention. The electronic module is enclosed in the closure cap 17 which is attached to the housing 2 or to the end cap 12 of an autoinjector. The PCB 16c is positioned on extension 12a and the proximal end of a guide sleeve 12b. The battery 16b is fixated to the PCB by battery holders 26 contacting the terminals 16d, 16e of the battery 16b. One of the battery holders 26, in this example the battery holder contacting the negative terminal 16e, includes a battery contact extension 27. The battery contact extension 27 and the battery holder 26 for the negative terminal are made as one unitary component from a stamped sheet of metal. The battery contact extension 27 includes a part that is oriented essentially parallel to the battery terminals and a part that is oriented essentially perpendicular to the battery terminal. A kink 27b is positioned between the parallel and perpendicular oriented parts. The part of the battery contact extension oriented perpendicular to the battery terminal provides for a detection pin 27c and the part parallel to the terminals provides for a leaf spring 27a. The PCB includes a passage 16f for the detection pin 27c. The distal end 27d of the detection pin 27c is guided by the guide sleeve 12b through a passage 12c in the end cap 12 towards the signaling element 11 of the autoinjector. Before use of the autoinjector, the signaling element 11 abuts the distal end 27d of the detection pin and pushes the detection pin 27c against the bias provided by the leaf spring 27a in the proximal direction thereby creating a switching gap between the battery contact extension 27 and the PCB 16c to open the switch that will be discussed in more detail below.

The electronic module of Figure 4a is presented in Figure 4b during use of the autoinjector. The signaling element 11 is driven in the distal direction by the injection spring during dose delivery. The distal end 27d of the detection pin 27c follows the distal movement due to the bias provided by the leaf spring 27a. The battery contact extension 27 contacts a contact that is part of an electronic circuit 29 located on the PCB 16c thereby closing the switch.

A comparison of Figure 3a and Figure 4a shows the effect of replacing the switch 16a according to the prior art by the battery contact extension 27 and the electric activation contact 28 of the current disclosure. Fewer parts are required for activating the module by actuating switch. Additionally, the space occupied by the switch 16a in Figure 3a is available for a larger battery having a greater capacity in Figure 4a.

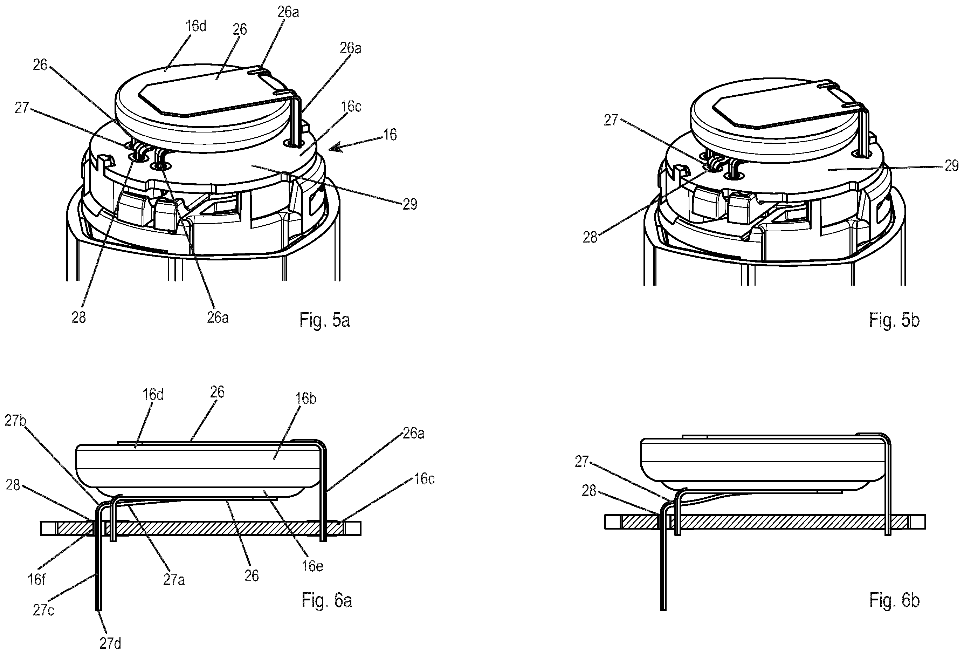

A perspective view of the electronic module 16 without the closure cap is presented in Figure 5a. The battery holder 26 contacts the positive terminal 16d (top surface of the battery) and extensions 26a are connected to the PCB 16c providing for mechanical support to the battery holder 26 and/or for the electric activation contact to the electronic circuit 29 on the PCB. Extensions 26a also extend from the battery holder contacting the negative terminal (bottom surface of the battery). In the embodiment presented in Figure 5a, two extensions 26a extend from the battery holders 26 to provide mechanical support for the battery and/or electric contacts to the electronic circuit 29 on the PCB. The battery contact extension 27 is located between the two extensions 26a for the battery holder contacting the negative terminal or bottom surface of the battery. The autoinjector is shown in Figure 5a before use and the battery contact extension 27 is moved in the proximal direction against the bias of the leaf spring such that the battery contact extension 27 is not contacting the electric activation contact 28 that is part of the electronic circuit 29, e.g. the switch (27, 28) is open. The autoinjector is shown in Figure 5b during use and the switch (27, 28) is closed.

A side view of the electronic module with an open switch is presented in Figure 6a and with a closed switch in Figure 6b. The two battery holders 26 with their extensions 26a position, and hold the battery 16b on top of the PCB 16c. The battery contact extension 27 extends from the negative battery terminal 16e. The kink 27b of the battery contact extension is located between the leaf spring 27a and the detection pin 27c. The PCB 16c includes the passage 16f for the detection pin 27c and the distal end 27d is configured for engaging the drive element of the autoinjector thereby pushing the battery contact extension 27 in the proximal direction against the bias of the leaf spring. The battery contact extension 27 does not contact the electric activation contact 28 in Figure 6a (switch open) and the switch is closed in Figure 6b.

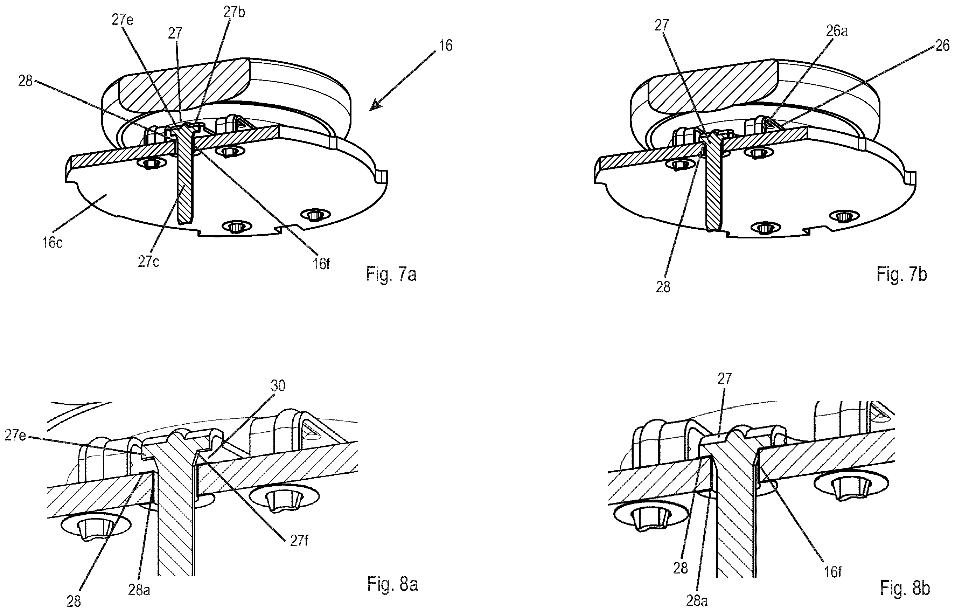

Perspective bottom views of the electronic module with sectional cuts are shown in Figures 7a and 7b and details in Figures 8a and 8b. The battery contact extension 27 is made from a stamped metal sheet with the detection pin 27c formed as a narrowed extension that is adjacent to the kink 27b thereby leaving a lateral extension 27e on one side of the passage 16f in the PCB as the detection pin 27c is guided through the passage 16f. A tapered section 27f depends from the lateral extension 27e (detail shown in Figure 8a). The electric activation contact 28 on the PCB surrounds the passage 16f and may contact the lateral extension 27e of the battery contact extension 27 for closing the switch 27, 28. The switch is open in Figures 7a and 8a as there is a switching gap 30 between the battery contact extension 27 and the electric activation contact 28. The switch is closed in Figures 7b and 8b and the lateral extension 27e of the battery contact extension 27 contact the electric activation contact 28. As an option, the electric activation contact may cover at least a part of the surface of the passage 16f itself. The electric activation contact in the passage 28a is configured for contacting the tapered section 27f. The tapered section may remove deposition or corrosion products from the surface of contacts 28, 28a when moving from an open switch (Figure 8a) to a closed switch (Figure 8b).

**PART ANNOTATION**

| | | | |
|---|---|---|---|
| 1 | Syringe holder | 15 | Switching sleeve |
| 1a | Arms | 15a | Distal end |
| 2 | Housing | 15b | Rim |
| 3 | Needle cover sleeve | 15c | Cut out |
| 3a | Proximal end | 16 | Electronic module |
| 3b | Recess | 16a | Prior art sensor/switch |
| 4 | Pen cap | 16b | Battery |
| 5 | Mechanic holder | 16c | Printed circuit board, PCB |
| 5c | Holding spring | 16d | Battery terminal (+) |
| 6 | Holding element | 16e | Battery terminal (-) |
| 6a | Central pin, guide pin | 16f | Passage PCB |
| 6b | Arms | 17 | Closure cap |
| 6c | Protrusion | 17a | Aperture |
| 6d | Proximal end | 18 | Pivoting element, detection |
| 7 | Piston rod | | pin |
| 7a | Recess | 19 | Bung |
| 7b | Recess | 20 | Auto injector |
| 8 | Locking sleeve | 21 | Prefilled syringe |
| 8a | Flexible arm | 21a | Proximal end |
| 9 | Injection spring | 22 | Needle shield |
| 9a | Distal end | 23 | Hollow needle |
| 9b | Proximal end | 24 | LED |
| 10 | Needle cover sleeve spring | 25 | Transparent plug |
| 10a | Distal end | 25a | Wings |
| 10b | Proximal end | 26 | Battery holder |
| 11 | Signaling element | 26a | Extension |
| 11a | Protrusion | 27 | Battery contact extension |
| 11b | Arms | 27a | Leaf spring |
| 11c | Protrusion | 27b | Kink |
| 12 | End cap | 27c | Detection pin |
| 12a | Protrusion / rim | 27d | Distal end |
| 12b | Guide sleeve | 27e | Lateral extension |
| 12c | Passage end cap | 27f | Tapered section |
| 14 | Needle shield remover | | |
| 28 | Electric activation contact PCB | 30 | Switching gap |
| | | L | Longitudinal axis |
| 28a | Electric activation contact passage | 8,15 | Switching module |
| | | 27,28 | Switch |
| 29 | Electronic circuit PCB | | |

## Claims

1. An electronic module configured for releasable attachment to a medicament delivery device (20) comprising
- a printed circuit board (16c) with an electronic circuit (29),
- a battery (16b),
- a switch for activating the electronic module (16),
**characterized in that** the switch comprises
- an electric activation contact (28) providing a connection with the electronic circuit (29),
- a battery contact extension (27) extending from a terminal (16d, 16e) of the battery (16b),
wherein the battery contact extension (27) is biased towards the electric activation contact (28) for closing a switching gap (30) between the battery contact extension (27) and the electric activation contact (28), and
wherein the battery contact extension (27) is configured to engage an actuation element (11,6) of the medicament delivery device (20) such that the actuation element (11, 6) pushes the battery contact extension (27) against the bias thereby creating the switching gap (30).

2. The electronic module according to claim 1 wherein the battery contact extension (27) comprises a spring element (27a) biasing the battery contact extension (27) towards the electric activation contact (28).

3. The electronic module according to claim 2 wherein the battery contact extension (27) comprises a detection pin (27c) and wherein the spring element (27a) is located between the terminal (16d,16e) of the battery (16b) and the detection pin (27c).

4. The electronic module according to claim 3 wherein the actuation element (11, 6) of the medicament delivery device (20) is configured to abut the detection pin (27c) of the battery contact extension (27).

5. The electronic module according to claim 4 wherein the detection pin (27c) and the spring element (27a) are provided as a unitary component made from an electrically conductive material.

6. The electronic module according to claim 5 wherein the spring element is a leaf spring (27a) and wherein the detection pin (27c) is oriented essentially perpendicular to the leaf spring (27a) forming a kink (27b) between the leaf spring (27a) and the detection pin (27c).

7. The electronic module according to claims 4 to 6 wherein the electric activation contact (28) at least partially surrounds a passage (16f) in the printed circuit board (16c).

8. The electronic module according to claim 7 wherein the detection pin (16c) at least partially passes through the passage (16f) and the distal end (27d) of the detection pin (27c) is configured to engage the actuation element (11, 6) of the medicament delivery device (20).

9. The electronic module according to claim 8 wherein the electric activation contact (28) at least partially covers the inner surface of the passage (16f).

10. The electronic module according to claim 9 wherein the battery contact extension (27) comprises a lateral extension (27e) and a tapered section (27f) for contacting the electric activation contact (28, 28a) wherein the lateral extension (27e) has an outer dimension greater than the inner dimension of the passage (16f).

11. The electronic module according to any of the previous claims wherein the actuation element (11,6) of the medicament delivery device (20) is configured to move axially along, or tilt with respect to, or rotate around a longitudinal axis of the medicament delivery device (20) when the medicament delivery device is used thereby releasing the engagement between the actuation element (11, 6) and the battery contact extension (27) to close the switching gap (30).

12. The electronic module according to any of the previous claims further comprising a mechanical interface configured for releasable attachment to the medicament delivery device (20).

13. The electronic module according to claim 12 wherein the detection pin (27c) is part of or located in the mechanical interface.

14. A medicament delivery device comprising the electronic module according to any of the previous claims attached to the medicament delivery device (20).

15. The medicament delivery device according to claim 14 wherein the medicament delivery device (20) is a spring driven auto injector and wherein an injection spring (9) moves a drive element (7) in the distal direction during dose delivery and rotates the actuation element (11,6) or tilts the actuation element (11,6) or axially moves the actuation element (11,6) in the distal direction to release the engagement between the battery contact extension (27c) and the actuation element (11,6).
